(19) Europäisches Patentamt
European Patent Office
Office européen des brevets

(11) Veröffentlichungsnummer: **0 445 552 A1**

# (12) EUROPÄISCHE PATENTANMELDUNG

(21) Anmeldenummer: **91101972.7**

(51) Int. Cl.[5]: **C07D 213/89**

(22) Anmeldetag: **13.02.91**

(30) Priorität: **09.03.90 DD 338543**

(43) Veröffentlichungstag der Anmeldung:
**11.09.91 Patentblatt 91/37**

(84) Benannte Vertragsstaaten:
**AT BE CH DE ES FR GB IT LI NL SE**

(71) Anmelder: **ARZNEIMITTELWERK DRESDEN GMBH**
**Wilhelm-Pieck-Strasse 35**
**O-8122 Radebeul(DE)**

(72) Erfinder: **Klauschenz, Erhard, Dr.**
**Roelcke-Strasse 12**
**O-1120 Berlin(DE)**
Erfinder: **Hagen, Volker, Dr.**
**Kuhlungsborner Strasse 29**
**O-1093 Berlin(DE)**
Erfinder: **Hagen, Angela, Dr.**
**Kuhlungsborner Strasse 29**
**O-1093 Berlin(DE)**
Erfinder: **Muschik, Peter, Dr.**
**Rathausstrasse 9**
**O-1020 Berlin(DE)**
Erfinder: **Schlegel, Brigitte**
**Zossener Strasse 97**
**O-1152 Berlin(DE)**
Erfinder: **Heer, Sabine, Dipl.-Biol.**
**Robinienstrasse 6**
**O-8023 Dresden(DE)**
Erfinder: **Faust, Gottfried, Dr.**
**Ernst-Thälmannstrasse 53**
**O-8122 Radebeul(DE)**
Erfinder: **Jänsch, Hans-Joachim, Dr.**
**Wilhelm-Pieck-Strasse 292**
**O-8122 Radebeul(DE)**

(74) Vertreter: **Puchberger, Rolf, Dipl. Ing. et al**
**Patentanwälte, Dipl. Ing. Georg Puchberger Dipl. Ing. Rolf Puchberger Dipl. Ing. Peter Puchberger Singerstrasse 13 Postfach 55**
**A-1010 Wien(AT)**

## (54) 3-Cyan-5,4'-oxide, Verfahren zu ihrer Herstellung und ihre pharmazeutische Verwendung.

(57)

3-Cyan-5,4'-bipyridin-1'-oxide, 3-Cyan-5,4'-bipyridine,2-Chlor-3-cyan-5,4'-bipyridin-1'-oxide, kardioton, vasodilatatorisch

3-Cyan-5,4'-bipyridin-1'-oxide der allgemeinen Formel I und ihre physiologisch verträglichen Säureadditionssalze besitzen wertvolle kardiotone und vasodilatatorische Eigenschaften. Sie können dadurch hergestellt werden, daß man entweder

a) 3-Cyan-5,4'-bipyridine der Formel II mit zur Erzeugung von Pyridin-N-oxiden geeigneten Reagenzien, vorzugsweise Persäuren oder Wasserstoffsuperoxid, umsetzt oder

b) 2-Chlor-3-cyan-5,4'-bipyridin-1'-oxide der Formel III mit einer Verbindung der Formel IV umsetzt

und die erhaltenen Verbindungen der Formel 1 erwünschtenfalls mit physiologisch verträglichen anorganischen oder organischen Säuren in ihre Säureadditionssalze überführt.

EP 0 445 552 A1

3-Cyan-5,4'-bipyridin-1'-oxide, Verfahren zu ihrer Herstellung und ihre pharmazeutische Verwendung

Die Erfindung betrifft 3-Cyan-5,4'-bipyridin-1'-oxide der allgemeinen Formel I, worin R für Amino, alkylsubstituiertes Amino. Aminoalkylamino, Oxaalkylamino, Alkoxy, Oxaalkoxy oder Chlor stehen, ihre physiologisch verträglichen Säureadditionssalze, Verfahren zu ihrer Herstellung und ihre pharmazeutische Verwendung.

Die Verbindungen der allgemeinen Formel I als auch ihre physiologisch verträglichen Säureadditionssalze besitzen wertvolle kardiotone und vasodilatatorische Eigenschaften und können deshalb zur Herstellung von Arzneimitteln für die Behandlung entsprechender Krankheiten eingesetzt werden.

Entsprechend der obigen Bedeutung für R wird unter alkylsubstituiertem Amino $C_{1-4}$ -Monoalkylamino oder $C_{1-4}$-Dialkylamino, unter Aminoalkylamino Amino-$C_{1-4}$-alkylamino, Di-($C_{1-4}$-alkyl)-amino-$C_{1-4}$-alkylamino, Morpholino-$C_{1-4}$-alkylamino oder gegebenenfalls durch $C_{1-4}$-Alkyl oder Hydroxy-$C_{1-4}$-alkyl substituiertes Piperazino, unter Oxaalkylamino Hydroxy-$C_{2-4}$-alkylamino, Di-(hydroxy-$C_{2-4}$-alkyl)-amino, $C_{1-4}$-Alkoxy-$C_{2-4}$-alkylamino oder Morpholino, unter Alkoxy $C_{1-4}$-Alkoxy, unter Oxaalkoxy Hydroxy-$C_{2-4}$-alkoxy oder Dihydroxy-$C_{3-4}$-alkoxy oder $C_{1-4}$-Alkoxy-$C_{2-4}$-alkoxy verstanden.

2-Amino- und 2-Alkylamino-3-cyan-5-pyridyl-pyridine (G. Y. Lesher u.a. US-PS 4 362 734, DD-PS 263 758 und 275 047, Die Pharmazie 44. 20 (1989) sowie 2-Aminoalkylamino-3-cyan-, 3-Cyan-2-oxaalkoxy und 3-Cyan-2-oxaalkylamino-5-(pyrid-4-yl)-pyridine bzw. 3-Cyan-2-morpholino-5-(pyrid-4-yl)-pyridin und ihre kardiotone Wirkung sind beschrieben (EP-PS 200 024 A2).

Ebenfalls bekannt sind 2-Chlor-3-cyan- und 2-Alkoxy-3-cyan-5-(pyrid-4-yl)-pyridin (siehe zum Beispiel G. Y. Lesher u.a., US-PS 4 264603, Pol . J. Pharmacol. 30, 707 (1978), US-PS 4 463 008, ES-PS 518 498) sowie ihre kardiotone Wirkung der letztgenannten Verbindung.

Biologisch wirksame Heterocyclen werden häufig auch in Form ihrer physiologisch verträglichen Säureadditionssalze, wie Hydrochloride, Sulfate, Acetate, Fumarate, Mesylate oder Tartrate verabreicht (DE-OS 26 37 600).

5,4'-Bipyridin-mono-1'-oxide mit funktionellen Gruppen in 2- und/oder 3-Position sind mit Ausnahme des 1'-Oxids von 3-Amino-5-(pyrid-4-yl)-pyrid-2-on (Amrinon und seines 3-Acetamido-Analogons bisher nicht synthetisiert worden. Für das 1'-Oxid des Amrinons wurden mäßige positiv inotrope Wirkungen angegeben (DE-OS 30 45 637). Erwähnt wurde das 1' -Oxid von 3-Cyan-6-methyl-5-(pyrid-4-yl)-1,2-dihydro-pyrid-2-on (Milrinon) als Metabolit dieses Wirkstoffes (Alouisi, A. A.; R. J. Fabian; J. F. Baker; R. M. Stroshane, New Drug Animal, Cardiovasc. Drugs 3, S. 269 (1985). Über eventuelle herzstimulierende Aktivitäten letzterer Verbindung wurde nichts berichtet.

Im Rahmen eigener Arbeiten, bei denen 3-Cyan-6-methyl-5-(pyrid-4-yl)-1,2-dihydropyrid-2-on-1'-oxid durch direkte N-Oxidation des Milrinons mittels Peressigsäure synthetisiert wurde, konnte festgestellt werden, daß die biologische Aktivität am Vorhof des Meerschweinchens infolge der N-Oxidation am Pyrid-4-yl-rest drastisch reduziert wird.

Der Erfindung liegt die Aufgabe zugrunde, biologisch, insbesondere kardioton und vasodilatatorisch, wirksame 3-Cyan-5,4'-bipyridin-1'-oxide der allgemeinen Formel I, worin R die oben genannten Bedeutungen besitzt und Verfahren zu ihrer Herstellung aufzufinden.

Entsprechend der vorliegenden Erfindung wird das dadurch erreicht, daß man entweder

a) 3-Cyan-5,4'-bipyridine der allgemeinen Formel II, worin R die oben genannte Bedeutung besitzt, mit zur Erzeugung von Pyridin-N-oxiden geeigneten Reagenzien, vorzugsweise Persäuren oder Wasserstoffsuperoxid, umsetzt oder

b) 2-Chlor-3-cyan-5,4'-bipyridin-1'-oxide der allgemeinen Formel III mit einer Verbindung der allgemeinen Formel IV, worin R die oben genannte Bedeutung besitzt, umsetzt

und die erhaltenen Verbindungen der allgemeinen Formel I erwünschtenfalls mit physiologisch verträglichen anorganischen oder organischen Säuren in ihre Säureadditionssalze überführt.

Eine besondere Ausführungsform der Erfindung besteht darin, daß man das Verfahren gemäß a) in Gegenwart eines inerten organischen Lösungsmittels wie Eisessig oder Chloroform und bei Temperaturen zwischen 10 und 100 °C, vorzugsweise zwischen 10 und 60 °C, durchführt, wobei es vorteilhaft sein kann, die Umsetzung durch weiteres Erwärmen, z. B. auf 70 bis 100 °C, zu vervollständigen.

Beispiele für Persäuren, die für die Durchführung des Verfahrens gemäß a) geeignet sind, sind Peressigsäure, Perameisensäure, Perbenzoesäure, m-Chlorperbenzoesäure oder Perphthalsäure.

Eine besondere Ausführungsform der Erfindung besteht darin, daß man das Verfahren gemäß b) in inerten organischen Lösungsmitteln wie tertiären Aminen, Pyridin, Methylglykol, Dioxan, Dimethylformamid, Dimethylsulfoxid, Acetonitril oder Gemischen dieser Lösungsmittel oder in einem Überschuß der eingesetzten Amine durchführt.

Wird die Umsetzung gemäß dem Verfahren b) in inerten organischen Lösungsmitteln durchgeführt,

besteht eine besondere Ausführungsform der Erfindung darin, daß die Umsetzung in Gegenwart von Säureacceptoren wie KOH, NaOH oder $K_2CO_3$ durchgeführt wird.

Auf Grund ihrer kardiotonen und vasodilatatorischen Eigenschaften können die Verbindungen der allgemeinen Formel I einzeln oder im Gemisch miteinander oder auch in Kombination mit anderen Wirkstoffen zu pharmazeutischen Mitteln verarbeitet werden, die je nach Applikationsform zusätzlich physiologisch verträgliche Träger- und/oder Hilfsstoffe enthalten können.

Diese pharmazeutischen Mittel sowie die Verfahren zu ihrer Herstellung sind ebenfalls Bestandteil dieser Erfindung.

Wie bereits ausgeführt, besitzen die Verbindungen der allgemeinen Formel I wertvolle kardiotone und vasodilatatorische Eigenschaften. Beispielsweise wurde für das 3-Cyan-2-morpholino-5,4'-bipyridin-1'-oxid eine hohe vasodilatatorische und positiv inotrope Wirkung in vitro und in vivo an verschiedenen Tiermodellen gefunden.

Dies ist besonders überraschend, da das von uns vergleichsweise hergestellte 1'-Oxid des Milrinons nur eine geringe kardiotone Aktivität aufweist, obwohl Milrinon selbst zu den am stärksten wirksamen nichtglykosidischen Kardiotonika zählt.

Die Erfindung wird im folgenden anhand von Ausführungsbeispielen näher erläutert.

Beispiel 1

3-Cyan-2-morpholino-5,4'-bipyridin-1'-oxid

Zu einer Lösung von 1,3 g 3-Cyan-2-morpholino-5-(pyrid-4-yl)-pyridinin 20 ml Chloroform gibt man unter Rühren bei Raumtemperatur 1,1 g m-Chlor-perbenzoesäure. Die Reaktionsmischung wird 48 Stunden bei Raumtemperatur gerührt. Anschließend wird mit Bikarbonatlösung extrahiert und die Chloroform-Phase im Vakuum eingeengt. Den Rückstand versetzt man mit Wasser, saugt ab, trocknet bei 110 °C und kristallisiert aus einem Gemsich aus Isopropanol und Ethanol um.
Ausbeute: 1,0 g (73 % der Theorie).
F.: 233 - 235 °C

Beispiel 2

3-Cyan-2-morpholino-5,4'-bipyridin-1'-oxid

Eine Mischung aus 10 g 2-Chlor-3-cyan-5,4'-bipyridin-1'-oxid und 40 ml Morpholin wird 10 Minuten auf 70 - 80 °C erwärmt. Anschließend engt man im Vakuum bei einer Badtemperatur von 80 °C bis zur Trockne ein, sammelt den Rückstand, wäscht sorgfältig mit Wasser und trocknet bei 110 °C. Dieses Produkt kocht man mit 150 ml Dichlormethan 1 Stunde am Rückfluß, saugt ab, trocknet abermals bei 110 °C und kristallisiert aus n-Butylacetat um.
Ausbeute: 9,27 g (75 % der Theorie).
F.: 234 - 236 °C.

Beispiel 3

3-Cyan-2-morpholino-5,4'-bipyridin-1'-oxid-hydrochlorid

Man löst 1 g 3-Cyan-2-morpholino-5,4'-bipyridin-1'-oxid in 5 ml 2H HCl und versetzt die Lösung unter Kühlung mit iso-Propanol und Ether, saugt ab und trocknet bei 100 °C.
Ausbeute: 0,9 g (79,8 % der Theorie)
F.: 222 - 223 °C.

Beispiel 4

3-Cyan-2-(3-diethylamino-1-propylamino)-5,4'-bipyridin-1'-oxid

Man erwärmt eine Mischung aus 1 g 2-Chlor-3-cyan-5,4'-bipyridin-1'-oxid und 1,35 g 3-Diethylamino-1-propylamin in 50 ml Ethanol 4 Stunden am Rückfluß. Anschließend engt man den Ansatz im Vakuum ein und kristallisiert das zurückbleibende Öl zunächst aus Ethanol/Diethylether und anschließend die dabei gewonnenen Kristalle aus Methanol um.

Ausbeute: 1.3 g (90,9 % der Theorie)
F.: 132 - 133 °C.

Beispiel 5:

2-Chlor-3-cyan-5,4'-bipyridin-1'-oxid

Eine Mischung aus 32.3 g 2-Chlor-3-cyan-5-(pyrid-4-yl)-pyridin und 245 ml Eisessig erwärmt man auf dem Wasserbad unter Rühren auf 70 °C und tropft vorsichtig 42 ml 40 %ige Peressigsäure hinzu. Es entsteht eine hellgelb gefärbte klare Lösung.

Nach dem Zutropfen steigert man die Temperatur des Wasserbades auf 80 °C unter fortwährendem Rühren. Anschließend tropft man in Intervallen von 1 Stunde nochmals 25 ml 40 %ige Peressigsäure in Portionen von je 5 ml (5 x 5 ml) in die Mischung. Nach 6 Stunden schüttet man den Ansatz auf Eis. saugt den Niederschlag ab, wäscht intensiv mit Wasser, kocht anschließend den Niederschlag in 500 ml Ethanol 1 Stunde am Rückfluß, saugt ab und trocknet.
Ausbeute: 33 g (95 % der Theorie).
F.: 261 - 263 °C.

Beispiel 6

3-Cyan-2-dimethylamino-5,4'-bipyridin-1'-oxid

3 g 2-Chlor-3-cyan-5,4'-bipyridin-1'-oxid und 3 g N,N-Dimethylammonium-N,N-dimethylcarbamat werden in 50 ml Dimethylformamid 2 Stunden auf dem siedenden Wasserbad erwärmt. Man läßt anschließend abkühlen und saugt nach Stehen über Nacht im Kühlschrank den entstandenen kristallinen Niederschlag ab, kristallisiert aus Dimethylformamid um und trocknet bei 110 °C.
Ausbeute: 2,9 g (93,2 % der Theorie).
F.: 282 - 284 °C.

Beispiel 7

3-Cyan-2-(2-hydroxy-ethylamino)-5,4'-bipyridin-1'-oxid

5 g 2-Chlor-3-cyan-5,4'-bipyridin-1'-oxid und 8 ml Ethanolamin werden in 50 ml Ethanol 4 Stunden am Rückfluß erwärmt. Nach Einengen des Ansatzes im Vakuum nimmt man den Rückstand in Wasser auf, saugt ab, wäscht mit Wasser, trocknet bei 110 °C und kristallisiert aus Ethanol/Eisessig unter Zusatz von Aktivkohle um.
Ausbeute: 4,5 g (81,3 % der Theorie)
F.: 229 - 231 °C.

Beispiel 8

3-Cyan-2-(3-hydroxy-propylamino)-5,4'-bipyridin-1'-oxid-hydrat

Analog Beispiel aus 5 g 2-Chlor-3-cyan-5,4'-bipyridin-1'-oxid und 8 ml 3-Amino-propanol-(1) und Umkristallisation aus Ethanol/Wasser.
Ausbeute: 4,87 g (78,4 % der Theorie).
F.: 141 - 143 °C.

Beispiel 9

3-Cyan-2-piperazino-5,4'-bipyridin-1'-oxid-hydrochlorid

Eine Mischung aus 2 g 2-Chlor-3-cyan-5,4'-bipyridin-1'-oxid, 0,8 g Piperazin, 0,52 g $KHCO_3$ und 80 ml Ethanol wird 3 Stunden am Rückfluß gekocht. Anschließend engt man im Vakuum bis zur Trockne ein, löst den Rückstand in 10 ml 1N HCl und versetzt die Lösung bis zur Kristallisation mit Ethanol. Nach Kühlung über Nacht saugt man die entstandenen Kristalle ab und trocknet bei 110 °C.
Ausbeute: 2,23 g (81,2 % der Theorie).

F.: 255 - 260 °C.

Beispiel 10

3-Cyan-2-morpholino-5,4'-bipyridin-1'-oxid-methansulfonat

Man löst 0,3 g 3-Cyan-2-morpholino-5,4'-bipyridin-1'-oxid unter Erwärmen in Aceton, läßt abkühlen, versetzt die Lösung mit 0,1 ml Methansulfonsäure, engt im Vakuum am Rotationsverdampfer ein, löst den öligen Rückstand in Aceton und versetzt die Lösung bis zur Kristallisation mit Ether, kühlt, saugt ab, wäscht mit Aceton und Ether und trocknet.
Ausbeute: 0,3 g (74,0 % der Theorie).
F.: 151 - 154 °C.

Beispiel 11

3-Cyan-2-(2,3-dihydroxy-propoxy)-5,4'-bipyridin-1'-oxid

300 mg KOH werden unter Erwärmen in 15 ml Glycerin gelöst, anschließend 1 g 2-Chlor-3-cyan-5,4'-bipyridin-1'-oxid zugefügt, 3 Stunden bei 80 - 90 °C gerührt, abkühlen gelassen, mit Wasser versetzt und der erhaltene Niederschlag abgesaugt.
Ausbeute: 2 g (89,5 % der Theorie)
F.: 194 - 196 °C

Beispiel 12

Isolierter, spontanschlagender Vorhof des Meerschweinchens

Die Versuche wurden wie von V. Hagen et al., Pharmazie 44, 20 (1989) beschrieben durchgeführt. Die substituierten 3-Cyan-5,4'-bipyridin-1'-oxide zeigten eine deutliche und konzentrationsabhängig positiv inotrope Wirkung.

Beispielsweise zeigen die Verbindungen der Beispiele 1, 4 und 8 eine 30 %ige Steigerung der Inotropie bei Konzentrationen von $2,9 \times 10^{-5}$ , $4,3 \times 10^{-4}$ bzw. $3,1 \times 10^{-4}$ mol/l. Auffällig sind die geringen Frequenzbeeinflussungen. Die Verbindung von Beispiel 4 zeigt sogar eine negative Chronotropie, was als besonders vorteilhaft angesehen werden kann. Die positiv inotrope Wirkung von Amrinon liegt vergleichsweise bei einer $ED_{30}$ bei $7,1 \times 10^{-4}$ mol/l.

Das 1'-Oxid von Milrinon zeigt bei $1,0 \times 10^{-5}$, $1,0 \times 10^{-4}$ bzw. $1,0 \times 10^{-3}$ mol/l, eine Inotropieerhöhung von lediglich 2,9 %, 8,4 % bzw. 6,5 %.

Beispiel 13

Narkotisierter Hund i. v.

Die Versuche wurden wie von V. Hagen et al., Pharmazie 44, 20 (1989) beschrieben durchgeführt.

Unter dem Einfluß der substituierten 3-Cyan-5,4'-bipyridin-1'-oxide wurde eine dosisabhängige deutliche Steigerung der Kontraktionskraft des Herzens sowie eine signifikante Senkung des peripheren Gesamtwiderstandes beobachtet.

So zeigen beispielsweise die Verbindungen der Beispiele 1 und 8 eine 50 %ige Steigerung des Kontraktilitätsparameters $dp/dt_{max}$ bei einer Dosis von $1,56 \times 10^{-7}$ bzw. $8,9 \times 10^{-6}$ mol/kg. Die Verbindung von Beispiel 1 zeigt eine 10 %ige Senkung des diastolischen Blutdruckes bei einer Dosis von $3 \times 10^{-6}$ mol/kg. Das vergleichsweise mitgeführte Standardpharmakon Amrinon zeigt demgegenüber eine positiv inotrope Wirkung mit einer $ED_{50}$ von $6,1 \times 10^{-6}$ mol/kg. Die $ED_{50}$ der Verbindung 3-Cyan-2-morpholino-5-(pyrid-4-yl)-pyridin beträgt an diesem Modell $1,6 \times 10^{-6}$ mol/kg.

Das 1'-Oxid von Milrinon zeigt bei den Dosierungen $4,4 \times 10^{-7}$, $2,2 \times 10^{-6}$ bzw. $2,2 \times 10^{-5}$ mol/kg lediglich eine Erhöhung des Kontraktilitätsparameters $dp/dt_{max}$ von 4 %, 12 % bzw. 39 %.

Beispiel 14

Narkotisiertes Minischwein i. v.

Die Untersuchungen wurden an 6 männlichen Zwergschweinen (Mini-LEWE) mit einem durchschnittlichen Gewicht von 50 kg durchgeführt. Die Tiere wurden unter $N_2O$-$O_2$-Beatmung (3 : 1) und Muskelrelaxation (d-Tubocurarin) thorakotomiert. Der Blutdruck wurde in der Aorta ascendens mittels Transducer und der Druck im linken Ventrikel (LVP) mit einem Tipmanometer gemessen. Aus dem LVP-Signal wurde über einen Integrator das Herz-Schlag-Volumen ermittelt.

Einige der 2-substituierten 3-Cyan-5,4'-bipyridin-1'-oxide der Formel I zeigen an diesem Modell eine starke und dosisabhängige Erhöhung der Kontraktilität des Herzens ($dp/dt_{max}$) sowie teilweise eine signifikante Senkung des totalen peripheren Gesamtwiderstandes. So wurde beispielsweise bei der Verbindung von Beispiel 1 bei einer Dosis von 2,8 x $10^{-7}$ mol/kg eine 50 %ige Steigerung des Kontraktilitätsparameters $dp/dt_{max}$ gefunden. Demgegenüber steigt unter Amrinon bzw. Milrinon der Kontraktilitätsparameter $dp/dt_{max}$ an diesem Modell nicht signifikant dosisabhängig und nur bis zu einem Maximalwert von 31,4 % bzw. 40,8 % bei einer Dosis von 8,5 x $10^{-6}$ mol/kg.

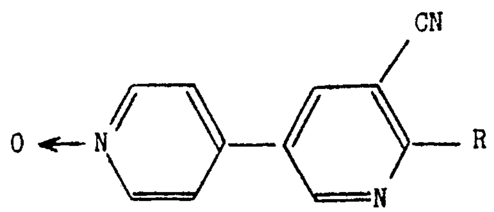

I

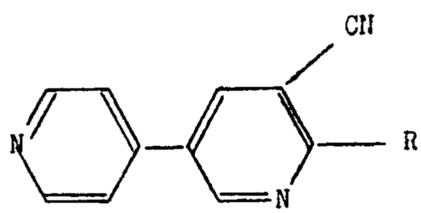

II

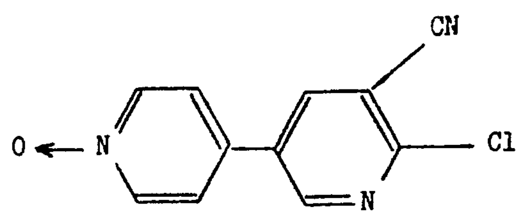

III

R - H

IV

**Patentansprüche**

1. 3-Cyan-5,4'-bipyridin-1'-oxide der Formel I,in der R für Amino, alkylsubstituiertes Amino, Aminoalkylamino, Oxaalkylamino, Alkoxy, Oxaalkoxy oder Chlor stehen und ihre physiologisch verträglichen Säureadditionssalze.

2. Verbindungen nach Anspruch 1, dadurch gekennzeichnet, daß R gleich alkylsubstituiertes Amino für $C_{1-4}$-Monoalkylamino oder $C_{1-4}$-Dialkylamino, daß R gleich Aminoalkylamino für Amino-$C_{1-4}$-alkylamino, Di-($C_{1-4}$-alkyl)-amino-$C_{1-4}$-alkylamino, Morpholino-$C_{1-4}$-alkylamino oder gegebenenfalls durch $C_{1-4}$-Alkyl oder Hydroxy-$C_{1-4}$-alkyl substituiertes Piperazino, daß R gleich Oxaalkylamino für Hydroxy-

$C_{2-4}$-alkylamino, Di-(hydroxy-$C_{2-4}$-alkyl)-amino, $C_{1-4}$-Alkoxy-$C_{2-4}$-alkylamino, Morpholino, daß R gleich Alkoxy für $C_{1-4}$-Alkoxy und daß R gleich Oxaalkoxy für Hydroxy-$C_{2-4}$-alkoxy oder Dihydroxy-$C_{3-4}$-alkoxy oder $C_{1-4}$-Alkoxy-$C_{2-4}$-alkoxy steht.

**3.** Verbindungen nach den Ansprüchen 1 und 2 dadurch gekennzeichnet, daß R Methylamino, Ethylamino, Dimethylamino, Diethylamino, 2-Hydroxyethylamino, 3-Hydroxypropylamino, Methoxy, Ethoxy, 2,3-Dihydroxy-propoxy, Morpholino, Piperazino oder Chlor bedeutet.

**4.** Verfahren zur Herstellung von 3-Cyan-5,4'-bipyridin-1'-oxiden der allgemeinen Formel I, worin R die in den Ansprüchen 1 bis 3 genannte Bedeutung besitzt dadurch gekennzeichnet, daß man entweder

a) 3-Cyan-5,4'-bipyridine der Formel II, worin R die in den Ansprüchen 1 bis 3 genannte Bedeutung besitzt, mit zur Erzeugung von Pyridin-N-Oxiden geeigneten Reagenzien, vorzugsweise Persäuren oder Wasserstoffsuperoxid umsetzt, oder

b) 2-Chlor-3-cyan-5,4'-bipyridin-1'-oxide der Formel III mit einer Verbindung der Formel IV worin R die in den Ansprüchen 1 bis 3 angegebene Bedeutung besitzt umsetzt

und die erhaltenen Verbindungen der Formel I erwünschtenfalls mit physiologisch verträglichen anorganischen oder organischen Säuren in ihre Säureadditionssalze überführt.

**5.** Verfahren nach Anspruch 4a, dadurch gekennzeichnet, daß als Persäure Peressigsäure Perameisensäure Perbenzoesäure m-Chlorperbenzoesäure oder Perphthalsäure eingesetzt werden

**6.** Verfahren nach den Ansprüchen 4a und 5 dadurch gekennzeichnet daß die Umsetzung in Gegenwart eines inerten organischen Lösungsmittels wie Eisessig oder Chloroform bei Temperaturen zwischen 10 und 100 °C vorzugsweise zwischen 10 und 60 °C, durchgeführt wird.

**7.** Verfahren nach Anspruch 4b, dadurch gekennzeichnet, daß die Umsetzung in inerten organischen Lösungsmitteln wie tertiären Aminen, Pyridin, Methylglykol, Dioxan, Dimethylformamid, Dimethylsulfoxid, Acetonitril oder Gemischen dieser Lösungsmittel oder in einem Überschuß der eingesetzten Amine selbst, durchgeführt wird.

**8.** Verfahren nach den Ansprüchen 4b und 7 dadurch gekennzeichnet, daß die Umsetzung in Gegenwart eines Säureacceptors wie KOH, NaOH, $K_2CO_3$ durchgeführt wird.

**9.** Pharmazeutische Mittel, dadurch gekennzeichnet, daß sie mindestens eine Verbindung der Formel I nach den Ansprüchen 1 bis 3 und physiologisch verträgliche Träger- und/oder Hilfsstoffe enthalten.

**10.** Verwendung von Verbindungen der Formel I nach den Ansprüchen 1 bis 3 zur Herstellung von Arzneimitteln mit kardiotonen und vasodilatatorischen Wirkungen.

**11.** Verwendung nach Anspruch 10, dadurch gekennzeichnet, daß die Verbindungen der allgemeinen Formel I allein oder in Kombination mit anderen Wirkstoffen eingesetzt werden.

**12.** Verfahren zur Herstellung von Arzneimitteln nach den Ansprüchen 9 bis 11, dadurch gekennzeichnet, daß die Verbindungen der Formel I nach den Ansprüchen 1 bis 3 allein oder in Kombination untereinander oder in Kombination mit anderen Wirkstoffen sowie gegebenenfalls pharmazeutischen Träger- und/oder Hilfsstoffen zu pharmazeutischen Zubereitungen verarbeitet werden.

Europäisches Patentamt

# EUROPÄISCHER RECHERCHENBERICHT

Nummer der Anmeldung

EP 91101972.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | DE - A1 - 3 045 637 (STERLING) * Ansprüche 1,2,4 * -- | 1,4,9 | C 07 D 213/89 |
| D,A | DD - A1 - 263 758 (AK. DER WISSENSCHAFTEN) * Zusammenfassung * -- | 1,4,9 | |
| A | GB - A - 1 542 605 (BRISTOL-MYERS) * Ansprüche 1,6,15 * ---- | 1,4,9 | |

RECHERCHIERTE SACHGEBIETE (Int Cl 5)

C 07 D 213/00

Der vorliegende Recherchenbericht wurde für alle Patentansprüche erstellt.

| Recherchenort | Abschlußdatum der Recherche | Prüfer |
|---|---|---|
| WIEN | 13-05-1991 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62